# EUROPÄISCHE PATENTANMELDUNG

(11) **EP 1 479 359 A1**
(43) Veröffentlichungstag der Anmeldung: **24.11.2004**
(21) Anmeldenummer: 03008239.0
(22) Anmeldetag: 09.04.2003
(51) Int. Cl.: A61F 2/40

(54) **Schultergelenkprothese**

(71) Anmelder: SQ Developments AG, 6312 Steinhausen (CH)
(72) Erfinder: Habermeyer, Peter, 69120 Heidelberg (DE); Kälin, Peter, 6314 Unterägeri (CH)
(74) Vertreter: Troesch Scheidegger Werner AG

(57) **Zusammenfassung**

Eine Schultergelenkprothese besteht mindestens aus einem Schaft (3), einem Winkelteil (13) und einem Kopfteil bzw. einem Gelenkkopf (19), wobei der Gelenkkopf eine teilkugelförmige Oberfläche aufweist mit einem Kugeldurchmesser, welcher kleiner ist als der entsprechende anatomische Kugeldurchmesser.

## Beschreibung

Die vorliegende Erfindung betrifft eine Total-Schultergelenk- bzw. Glenohumeralprothese gemäss dem Oberbegriff nach Anspruch 1.

Im Speziellen betrifft die vorliegende Erfindung eine Schulterprothese hauptsächlich für den Einsatz bei Patienten mit inoperablen Rotatorenmanschettendefekten und begleitendem Gelenkflächenverschleiss.

Rotatorenmanschettendefekte führen bekanntlich dazu, dass durch den Verlust der Zentrierung des Humeruskopfes in die Gelenkpfanne der Oberarmkopf nach oben auswandert, was den Hebelarm für den Deltamuskel verkürzt und damit die Abduktionskraft vermindert.

Es wurde deshalb versucht, diesen Verlust an Abduktionskraft durch Versetzung des Rotationszentrums des Schultergelenkes in Richtung zum Körper hin, d.h. durch ein medialisiertes Rotationszentrum des Kopfes zu kompensieren, da sich dadurch ein besseres Hebelarmverhältnis einstellt.

Entsprechend wird in der EP 0 299 889 eine Schultergelenkprothese vorgeschlagen, bei welcher der Gelenkkopf am Schulterblatt angeordnet wird und der Pfannenersatz entsprechend am Oberarmknochen. Dieses invers ausgebildete Implantat bewirkt wohl eine mediale Verschiebung des Rotationszentrums, führt aber zu einer Einschränkung des Bewegungsumfanges. Auch hat es sich gezeigt, dass die Übertragung von Schlägen relativ ungedämpft erfolgt und der Knochensubstanzverlust beim Implantieren der Prothese gemäss EP 0 299 889 erheblich ist. Es ist daher eine Aufgabe der vorliegenden Erfindung, eine Schultergelenkprothese zu schaffen, welche ein medialisiertes Rotationszentrum des Kopfes aufweist und welche weitgehenst keine oder nur eine reduzierte Einschränkung des Bewegungsumfanges mit sich bringt.

Erfindungsgemäss wird die gestellte Aufgabe mittels einer Total-Schultergelenkprothese gemäss dem Wortlaut nach Anspruch 1 gelöst.

Erfindungsgemäss wird im Gegensatz zur inversen Ausführung in der EP 0 299 889 eine "traditionelle" Schultergelenkprothese vorgeschlagen, mit dem Gelenkkopf am Oberarm angeordnet. Dabei weist die Prothese nebst Schaft und Winkelteil ein Kopfteil auf, welches einen Kugeldurchmesser aufweist, welcher im Vergleich zum anatomischen Kopfdurchmesser kleiner ausgebildet ist.

Vorzugsweise ist der Gelenkkopf mindestens als Halbkugel ausgebildet, wobei der Kugeldurchmesser vorzugsweise mehr als 20% kleiner ist als derjenige des anatomischen Oberarmkopfes. Die Verkleinerung des Kugeldurchmessers im Vergleich zum anatomischen Kopfdurchmesser bringt die Medialisierung des Kugelzentrums mit sich, was, wie eingangs erwähnt, ein besseres Hebelarmverhältnis ergibt. Allerdings bringt die erfindungsgemässe Ausbildung des Humeruskopfes mit sich, dass ein Ersatz lediglich alleine des Humeruskopfes auszuschliessen ist. Aus diesem Grund ist in jedem Fall ein Glenoidersatz notwendig. Somit wird das Implantat als Totalprothesensystem angewandt.

Das vorgesehene Glenoidteil bzw. die Pfanne ist vorzugsweise mindestens zweiteilig ausgebildet, mindestens aufweisend ein Konusteil und mindestens ein Befestigungsteil, vorzugsweise in Form einer Hohlschraube für die Verankerung des Glenoidteils in der Scapula. Die eigentliche Pfanne für die Aufnahme des Gelenkkopfes ist vorzugsweise aus einem hochverschleissfesten Polymerwerkstoff, wie beispielsweise aus Polyethylen mit ultrahohem Molkulargewicht(UHMW-PE) hergestellt. Die Metallteile des Glenoidteils, wie übrigens auch der Schaft der Humeruskopfprothese sind vorzugsweise aus Titanlegierungen hergestellt, der Gelenkkopf selbst vorzugsweise aus Kobaltchromlegierung.

Gemäss einer bevorzugten Ausführungsvariante sind der Kugeldurchmesser sowie die konkav ausgebildete Pfanne des Glenoidteils derart dimensioniert, dass sich eine wenigstens nahezu formschlüssige Verbindung zwischen Kopfteil und Pfanne innerhalb eines Winkelbereiches von mindestens ca. ± 45° ergibt. Selbstverständlich kann der formschlüssige Bereich kleiner oder grösser ausgebildet sein, doch hat es sich als vorteilhaft erwiesen, den formschlüssigen Bereich nicht wesentlich vergrössert auszubilden, um beispielsweise das Dämpfen von Schlägen zuzulassen.

Das erfindungsgemäss vorgeschlagene Implantat bringt beispielsweise im Vergleich zur Schultergelenkprothese gemäss EP 0 299 889 die folgenden Vorteile mit sich:
- relativ einfache und bewährte Operationstechnik
- bewährtes Schaftsystem für die dauerhafte Verankerung im Humerus
- verschiedene Halslängen des Gelenkkopfes möglich (variable Vorspannung)
- keine Einschränkung des Bewegungsumfanges
- bewährte Verankerung der Glenoidkomponente
- nur beschränkte Formschlüssigkeit Gelenkkopf-Pfanne
- gedämpfte Übertragung von Schlägen auf das Glenoid
- durch den geöffneten Pfannenradius wird ein physiologischer Roll-Gleitvorgang des Humeruskopfes ermöglicht. Dies reduziert den Pfannenverschleiss.

Die Erfindung wird nun beispielsweise und unter Bezug auf die beigefügten Figuren näher erläutert.

Dabei zeigen:
- Fig. 1 und 2: eine erfindungsgemässe Humeruskopfprothese in der Hauptansicht,
- Fig. 3: das vollständige Implantat inkl. Glenoidersatz im Längsschnitt,
- Fig. 4: die gleichzeitige Darstellung einer herkömmlichen Schultergelenkprothese und einer erfindungsgemässen Schultergelenkprothese, welche zwei Ausführungen mit unterschiedlichen Rotationszentren aufweist, und
- Fig. 5: in auseinander gezogener Darstellung und in Perspektive ein Glenoidteil der erfindungsgemässen Schultergelenkprothese.

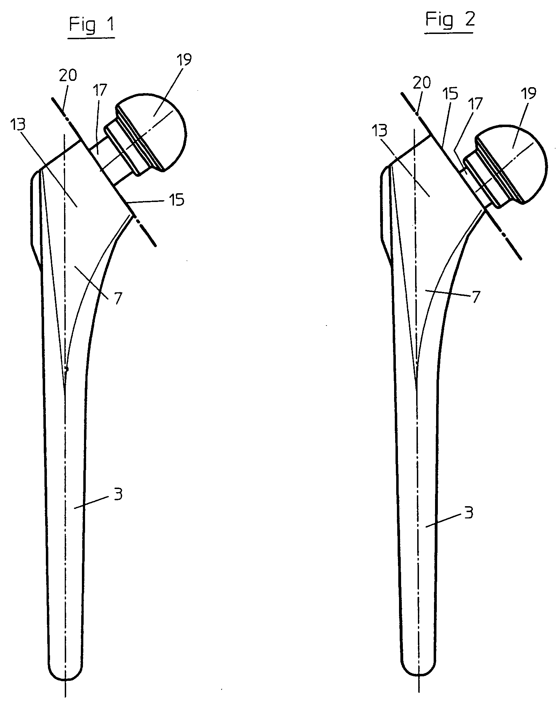

In den Fig. 1 und 2 ist eine erfindungsgemässe Humeruskopfprothese dargestellt, umfassend das untere, längs ausgebildete Einsteckteil bzw. den Schaft 3 sowie eine obere Partie 7, an welchem anschliessend das Winkelteil 13 ausgebildet ist. An einer Stirnfläche 15 des Winkelteils 13 ist der Gelenkkopf 19 auf einem Halsteil 17 angeordnet. Dabei zeigen Fig. 1 und 2 unterschiedliche Positionen des Gelenkkopfes 19.

Erfindungswesentlich ist nun, dass der Gelenkkopf relativ klein ausgebildet ist bzw. einen Kugeldurchmesser aufweist, welcher kleiner ist als der Durchmesser des anatomischen Humeruskopfes. Der erfindungsgemäss ausgebildete Gelenkkopf 19 kann dabei wie in den Fig. 1 und 2 halbkugelartig ausgebildet sein oder im Vergleich zu einer Halbkugel leicht grösser oder leicht kleiner ausgebildet sein. Wesentlich ist, dass der Kugeldurchmesser kleiner ist im Vergleich zum entsprechenden anatomischen Kopfdurchmesser, wobei der Durchmesser vorzugsweise mindestens 20% kleiner ist, vorzugsweise ca. 50 - 70% desjenigen des anatomischen Kopfes beträgt.

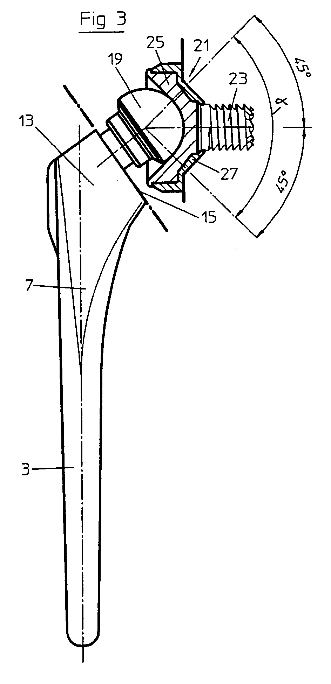

In Fig. 3 ist die gesamte Schulterprothese dargestellt, aufweisend die Humeruskopfprothese inkl. Gelenkkopf 19 wie auch den Glenoidersatz 21, bestehend aus Gelenkpfanne 25 und Konusteil 27, welcher vorzugsweise mittels einer Hohlschraube 23 in der Scapula verankert ist. Durch die Möglichkeit des unterschiedlichen Positionierens des Gelenkkopfes 19 auf der Stirnfläche 15 ist es möglich, gegebenenfalls verschiedene Ausführungen für spezielle anatomische Verhältnisse anzubieten. Die Ausbildung des Gelenkkopfes 19, aufweisend einen kleineren Durchmesser als der entsprechende anatomische Kopfdurchmesser, bringt eine mediale Verschiebung des Rationszentrums mit sich, wodurch beim Anheben des Armes eine wesentlich bessere Hebelwirkung erzielt werden kann, was insbesondere wichtig ist bei einem Verlust an Abduktionskraft.

Wie weiter aus Fig. 3 erkennbar, ist die Ausbildung des Glenoidteils nicht absolut formschlüssig, was die Dämpfung von Schlägen zulässt. Eine formschlüssige Verbindung zwischen dem Humeruskopf 19 und der Gelenkpfanne 25 ist vorzugsweise lediglich in einem Winkelbereich von ± 45° vorgesehen, was insgesamt einen formschlüssigen Bereich von ca. 90° ergibt. Selbstverständlich handelt es sich hierbei lediglich um ein Beispiel, grundsätzlich wesentlich ist, dass die Gelenkpfanne 25 nicht in ihrer Gesamtheit formschlüssig am Humeruskopf 19 anliegt. Der formschlüssige Bereich sollte aber auch einen Winkel von ca. 80° nicht unterschreiten, um eine ausreichende Stabilität zu gewährleisten.

Für die Herstellung der einzelnen Teile eignen sich an sich im Prothesenbau üblich verwendete Materialien. So ist es vorteilhaft, für Metallteile, wie den Schaft, das Konusteil, die Stirnfläche, etc. allgemein üblich verwendete Titaniumlegierungen zu verwenden, währenddem sich für die Herstellung der Gelenkpfanne im Glenoidteil speziell abriebfeste Polymermaterialien, wie beispielsweise UHMW-PE (Polyethylen mit ultrahohem Molekulargewicht) eignen. Diese ebenfalls im Prothesenbau an sich bekannten Polymere zeichnen sich durch extreme Schlag- und Verschleissfestigkeit aus sowie sehr guten Gleiteigenschaften.

Für die Oberfläche des Gelenkkopfes 19 werden ebenfalls vorzugsweise Metalllegierungen gewählt, welche über gute Gleiteigenschaften verfügen, wie beispielsweise Legierungen auf Kobaltchrombasis.

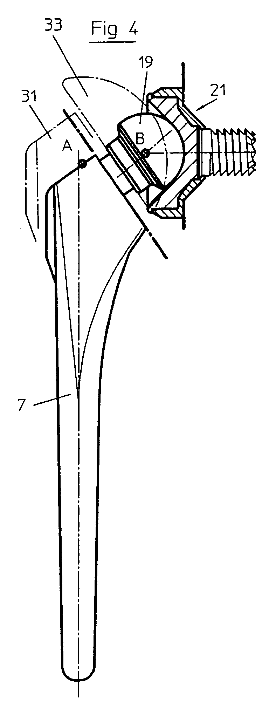

Fig. 4 zeigt die mediale Verschiebung des Rotationszentrums einer erfindungsgemässen Schultergelenkprothese im Vergleich zu einer traditionellen, herkömmlichen Prothese. In Fig. 4 gestrichelt eingezeichnet ist eine herkömmliche Prothese, aufweisend ein Schaft- und Winkelteil 31 sowie einen Gelenk- bzw. Humeruskopf 33. Mit A ist die Position des entsprechenden Rotationszentrums eingezeichnet.

Die erfindungsgemäss ausgebildete Schultergelenkprothese, weisend ein Schaftteil 7, einen Gelenkkopf 19 sowie ein Glenoidteil 21 mit Gelenkpfanne 25, weist ein Drehzentrum bzw. Rotationszentrum bei B auf. Die mediale Verschiebung des Rotationszentrums B in Bezug auf das herkömmliche Zentrum A ist deutlich erkennbar in Fig. 4.

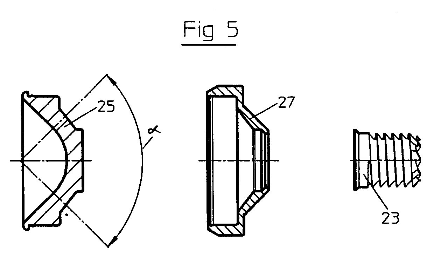

In Fig. 5 schliesslich zeigt das Glenoidteil 21 in auseinander gezogenem Zustand, d.h. zerlegt in Einzelteile. Die Gelenkpfanne 25, bestehend beispielsweise aus einem Polyethylenmaterial ist vorgesehen, um in einem Konusteil 27 eingeschnappt zu werden. Mittels Konusteil 27 und Hohlschraube 23 wird die Gelenkpfanne 25 in der Scapula bzw. dem Schulterblattknochen befestigt.

In Fig. 5 deutlich erkennbar ist, dass die Gelenkpfanne 25 nur in einem Bereich von ca. 90° gleichmässig gekrümmt ausgebildet ist, um formschlüssig am Humeruskopf der Humeruskopfprothese anzuliegen. Auf den Grund dieser Konstruktion wurde bereits unter Bezug auf Fig. 3 näher eingegangen.

Bei den in den Figuren 1 bis 5 dargestellten Schultergelenkprothesen sowie Einzelteile handelt es sich selbstverständlich um Ausführungsbeispiele und die vorliegende Erfindung ist keinesfalls auf diese Beispiele eingeschränkt. So können selbstverständlich Dimensionen der Einzelteile, wie insbesondere des Humeruskopfes selbst und der dazu gehörigen Gelenkpfanne abgeändert bzw. variiert werden, und auch die für die Prothese verwendeten Materialien können entsprechend den Bedürfnissen und auch Neuentwicklungen in den Materialwissenschaften entsprechend angepasst werden.

## Patentansprüche

1. Schultergelenkprothese mindestens bestehend aus einem Schaft (3), einem Winkelteil (13) und einem Kopfteil bzw. einem Gelenkkopf (19), **dadurch gekennzeichnet, dass** der Gelenkkopf eine teilkugelförmige Oberfläche aufweist mit einem Kugeldurchmesser, welcher kleiner ist als der entsprechende anatomische Kugeldurchmesser.

2. Prothese nach Anspruch 1, **dadurch gekennzeichnet, dass** der Gelenkkopf (19) unterschiedlich positioniert mit dem Winkelteil (13) verbunden ist.

3. Prothese nach einem der Ansprüche 1 oder 2, **dadurch gekennzeichnet, dass** der Gelenkkopf (19) im Wesentlichen als Halbkugel ausgebildet ist.

4. Prothese nach einem der Ansprüche 1 bis 3, **dadurch gekennzeichnet, dass** der Winkel zwischen Schaft und Achse des Kopfteils bzw. eines Kopfhalsteiles einen Winkel von 40° bis 55° einschliesst.

5. Prothese nach einem der Ansprüche 1 bis 4, **dadurch gekennzeichnet, dass** der Kugeldurchmesser des Gelenkkopfes um mehr als 20% kleiner ist als der Durchmesser des anatomischen Gelenkkopfes.

6. Prothese nach einem der Ansprüche 1 bis 5, **dadurch gekennzeichnet, dass** der Gelenkkopf im Wesentlichen als Halbkugel ausgebildet ist mit einem Durchmesser, welcher ca. 50 - 70% beträgt im Vergleich zum anatomischen Kugeldurchmesser.

7. Prothese nach einem der Ansprüche 1 bis 6, **dadurch gekennzeichnet, dass** weiter eine Pfanne bzw. ein Glenoidteil (21) vorgesehen ist, welche bzw. welches derart konkav angepasst an die Oberfläche des Gelenkkopfes (19), bzw. eine konusartige Vertiefung aufweisend ausgebildet ist, dass sich eine wenigstens nahezu formschlüssige Verbindung zwischen Kopfteil und Glenoidteil entlang mindestens eines Abschnittes der Pfanne bzw. des Kopfteils einstellt.

8. Prothese nach Anspruch 7, **dadurch gekennzeichnet, dass** die Pfanne und das Kopfteil bzw. der Gelenkkopf derart ausgebildet sind, dass sich eine wenigstens nahezu formschlüssige Verbindung innerhalb eines Winkelbereiches von mindestens ca. ± 40° (α = 80°) bis maximal ca. ± 70° (α = 140°), vorzugsweise ca. ± 45° (α = 90°) einstellt.

9. Prothese nach einem der Ansprüche 7 oder 8, **dadurch gekennzeichnet, dass** der Kugelradius der Pfanne bzw. des Glenoidteils insgesamt grösser ist als der Radius des Kopfteils bzw. des Gelenkkopfes.

10. Prothese nach einem der Ansprüche 7 bis 9, **dadurch gekennzeichnet, dass** mindestens ein Glenoidteil (21) vorgesehen ist, welches mindestens zweiteilig, vorzugsweise dreiteilig, ausgebildet ist, mindestens aufweisend eine Pfanne (25), vorzugsweise gehalten in einem Konusteil (27) und mindestens ein Befestigungsteil (23), vorzugsweise in Form einer Hohlschraube für die Verankerung des Glenoidteils in der Scapula.

11. Prothese nach einem der Ansprüche 1 bis 10, **dadurch gekennzeichnet, dass** der Gelenkkopf (19) zur Bildung eines kraftschlüssigen Gelenkes in einer Pfanne eines Glenoidteils (21) anliegt, wobei die Oberfläche der Pfanne des Glenoidteils aus einem polymeren Material besteht, welches extrem schlag- und verschleissfest ist und sehr gute Gleiteigenschaften aufweist, wie beispielsweise sogenanntes Polyethylen mit ultrahohem Molekulargewicht (UHMW-PE).
